# EUROPEAN PATENT APPLICATION

(11) **EP 4 529 909 A1**
(43) Date of publication of application: **02.04.2025**
(21) Application number: 24202705.0
(22) Date of filing: 25.09.2024
(51) Int. Cl.: A61F 15/00

(54) **FEMININE PRODUCT DISPENSING SYSTEM**

(30) Priority: 26.09.2023 US 202363585258 P
(71) Applicant: Cintas Corporate Services, Inc., Cincinnati, OH 45040 (US)
(72) Inventor: BING, Richard, West Chester, OH 45069 (US); HELMS, Megan Kathryn, Hamilton, OH 45013 (US); MESKO, David Stephen, Wyoming, OH 45215 (US); PERRY, Marco, Brooklyn, NY 11231 (US); YEAGER, Emily, Ft. Thomas, KY 41075 (US)
(74) Representative: Reddie & Grose LLP

(57) **Abstract**

A dispenser for personal care products includes one or more dispensing modules contained within a single housing. The dispenser may contain dispensing modules that independently dispense two distinct types of products. Products are packaged individually and dispensed accordingly in response to a user request. A feminine hygiene product dispenser includes multiple compartments which each contain a quantity of different feminine hygiene products. This dispenser may be located within a conventional toilet facility so that the cabinet may be located against a wall within the toilet facility regardless of variances in the installation setting.

## Description

### Background of the Invention

This claims the benefit of US Provisional Patent Application Serial No. 63/585,258 filed September 26, 2023 and hereby incorporated by reference in its entirety.

This invention relates to a feminine product dispensing system and associated method, and more particularly, to such a system which dispenses multiple kinds of products, minimizes physical contact with the dispensing system and viewing of the products.

Dispensing of feminine hygiene products, such as sanitary napkins, tampons, pads, and the like in a safe, sanitary, and discrete manner is often difficult. This issue may be particularly problematic in public restrooms which may not be equipped with proper dispensing equipment.

There has long been a need to construct some form of cabinet which is designed particularly to store feminine hygiene articles and have the cabinet designed where it is readily adaptable to each and every bathroom, public or private, and can be easily and quickly mountable within that bathroom.

Personal care products may be used for personal hygiene. For example, menstrual products can be used for personal hygiene during menstruation or other bodily functions. Common personal care products include menstrual products such as tampons and pads. The need for personal care products may come unexpectedly. A lack of readily available personal care products can negatively affect personal health and self-esteem, may cause soiling of clothing, and may disrupt daily activities. Conventionally, to obtain personal care products, an individual has to travel to a store or to their residence.

Conventional personal care product dispensing systems use products that are individually packaged with robust packaging to withstand forces during transportation, loading of the dispensing system, dispensing of the product, and the like. For example, each individual product, such as a tampon or pad, may be packaged in a box. The robust individual packaging of each product requires extra room within the dispensing system and increases costs of each product and of the dispensing system. This often results in the need for a larger dispensing unit that can hold a sufficient number of products containing the robust packaging. When used, the individual packaging around each personal care product, including any robust packaging, is typically discarded.

Determining when to restock a conventional dispensing system may also be difficult and time consuming. Conventionally, dispensing systems require that one open the dispenser to view how much inventory is left. Accordingly, the depletion or absence of stock may often go unnoticed. It is very common for conventional dispensers to remain empty for long periods of time because they do not hold a sufficient amount of product, they are time consuming to refill, or it is otherwise not apparent when the dispenser is empty.

In view of these deficiencies, special dispensers have been designed to alleviate at least some of the above-mentioned problems. However, many such dispensers do not provide a secure and controlled environment for the delivery of the hygiene products.

Therefore, there is a need in the art for an easy-to-use feminine hygiene storage container and dispenser which allows for sanitary dispensing of a variety of feminine hygiene products. Also, such a dispenser which is mountable and functional in a variety of settings is also desirable.

Various aspects of this invention address these particular needs and other needs, as will be disclosed in more detail below.

### Summary of the Invention

These and other objectives of this invention have been attained by various embodiments of this invention in which the dispenser offers convenient installation and intuitive operation.

The embodiments of this invention include a cabinet housing which includes a number of compartments. Each compartment has been specifically designed to facilitate the storing of a quantity of a particular type of feminine hygiene article, such as one compartment for external sanitary napkins, and another compartment for internal sanitary napkins. This dispenser has been designed to be installable in any one of various manners depending on the particular desired installation for a particular bathroom. The various mounting positions are provided to allow for each specific installation requirement.

One objective of this invention is to mount a feminine hygiene dispenser which can be quickly and easily installed in almost any bathroom with the feminine hygiene cabinet being an attractive addition to the bathroom at the same time making available to any woman that enters the bathroom various feminine hygiene articles.

The dispenser in various embodiments limits visual access to the interior of the dispenser and disposed products contained therein while maintaining optimized access to the interior. The disposal dispenser of various embodiments of this invention reduces visual and physical contact while integrating intuitive and familiar process operation steps.

### Brief Description of the Drawings

The above-mentioned and other features and advantages of this invention, and the manner of attaining them, will become more apparent and the invention itself will be better understood by reference to the following description of embodiments of the invention taken in conjunction with the accompanying drawings, wherein:
Fig. 1 is a perspective view of one embodiment of a dispenser according to this invention;
Fig. 2 is an exploded view of the dispenser of Fig. 1;
Fig. 3 is an enlarged view of hinge rods connecting a door to a housing of the embodiment of Fig. 2;
Fig. 4 is a perspective view of a pad module in the embodiment of Figs. 1-3;
Fig. 5 is a perspective view of a tampon module in the embodiment of Figs. 1-3;
Fig. 6 is a top view of a portion of the dispenser of Figs. 1-3 showing a first joint between the door and the housing;
Fig. 6A is an enlarged cross-sectional view taken along line 6A-6A of Fig. 6 of the hinge assembly in a locked configuration;
Fig. 6B is an enlarged cross-sectional view taken along line 6B-6B of Fig. 6A;
Figs. 7A and 7B are views similar to Figs. 6A-6B of the hinge assembly in an unlocked configuration;
Fig. 8 is a top view of the door swinging in a first direction from the housing to a first open position and in phantom lines in a second open position;
Fig. 9 is a perspective view of the dispenser with the door in the first open position;
Fig. 10 is a side elevational view in partial cross-section of the pad module in the dispenser;
Fig. 10A is a view similar to Fig. 10 with the pad module shown in cross-section;
Fig. 11 is a view similar to Fig. 10 with a pad being dispensed;
Fig. 11A is a view similar to Fig. 10A with the pad being dispensed;
Fig. 12 is a side elevational view in partial cross-section of the tampon module in the dispenser;
Fig. 12A is a view similar to Fig. 12 with a tampon being dispensed; and
Fig. 12B is a view similar to Fig. 12A with the tampon module in cross-section.

### Detailed Description of the Invention

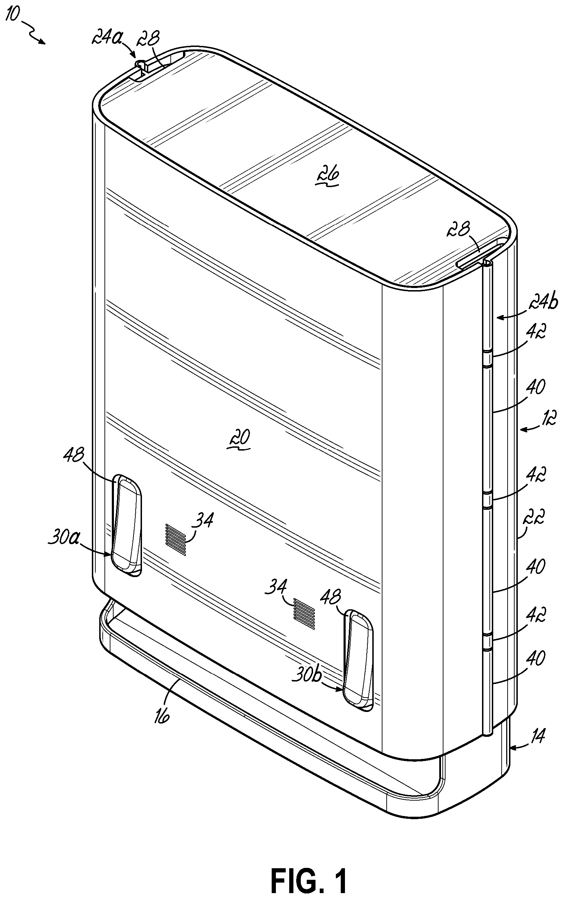

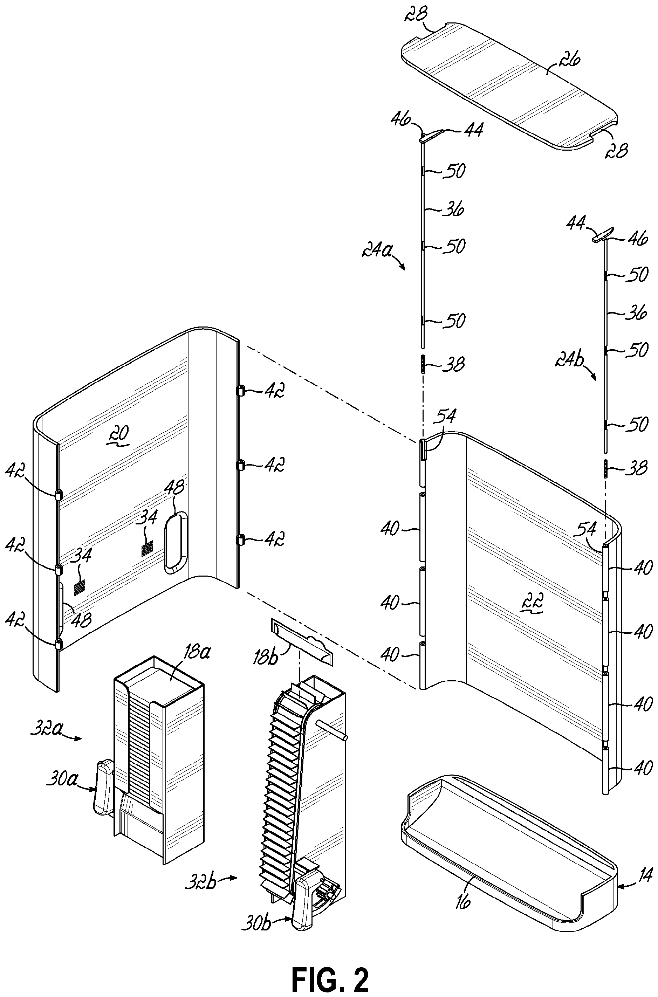

Referring to the drawings, an embodiment of a feminine product dispenser 10 is shown in Fig. 1 according to this invention. The dispenser 10 includes a housing 12 and a product retrieval tray 14 with a rim 16 below the housing 12 to receive feminine hygiene products 18a, 18b (see Fig. 2) dispensed from the dispenser 10. The housing 12 also includes a front door 20 coupled to a back body 22 by multiple hinge assemblies 24a, 24b. In the embodiment of Fig. 1, a first hinge assembly 24a and a second hinge assembly 24b are on opposite sides of the dispenser 10. The housing 12 also includes a top panel 26 with an access portal 28 on each end thereof for actuating the hinge assemblies 24a, 24b as will be explained herein below. A pair of buttons 30a, 30b project forwardly from the door 20. Each of the buttons 30a, 30b is coupled to one of two dispensing modules 32a, 32b contained within the housing 12 and are presented to a user to depress the buttons 30a, 30b and dispense the selected product 18a, 18b onto the tray 14 from the associated module 32a, 32b. A pair of viewing windows 34 are found in the door 20 through which a user may view the level of the product 18a, 18b in the associated module 32a, 32b. It will be appreciated by one of ordinary skill in the art that the dispenser, housing, tray, buttons, door, hinge assemblies, top panel, viewing windows and other components each may be of another configuration and/or arrangement in various embodiments of this invention all within the scope of this invention.

Referring to Fig. 2, each hinge assembly 24a, 24b according to one embodiment includes a hinge rod 36 and a hinge spring 38 and the back body 22 may include a number of spaced hinge barrels 40 along each terminal side edge thereof. The door 12 may also include a number of spaced hinge collars 42 along each terminal side edge thereof. When the door 12 is mated with the back body 22 as shown in Fig. 1, the hinge collars 42 are seated in the spaces between the hinge barrels 40 on each respective side edge thereof such that the associated hinge rod 36 may be inserted through the barrels 40 and aligned collars 42. Each hinge rod 36 may include an actuating member 44 at a top end thereof which is accessible to a user through the associated access portal 28 in the top panel 26 when the dispenser 10 is assembled. Each actuating member 44 may include a connecting arm 46 joining the actuating member 44 to the top end of the hinge rod 36.

As shown in Fig. 2, the dispenser 10 may include multiple dispensing modules 32a, 32b therein to dispense the same or different products 18a, 18b. In the embodiment shown in Fig. 2, the dispenser 10 includes a first dispensing module 32a for pads 18a and a second dispensing module 32b for tampons 18b. Each dispensing module 32a, 32b includes a button 30a, 30b respectively which projects through a button hole 48 in the front door 20 of the assembled dispenser 10 to actuate the associated dispensing module 32a, 32b.

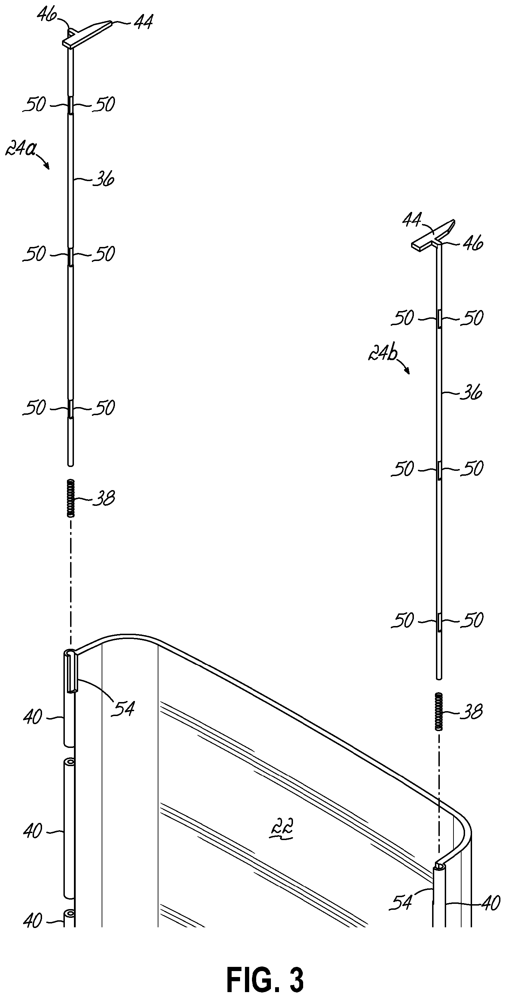

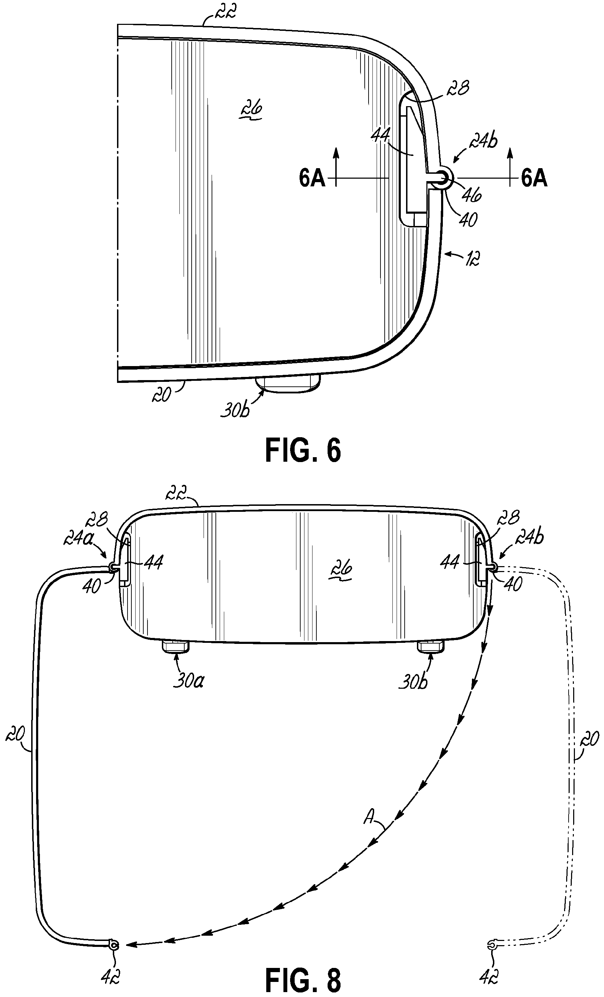

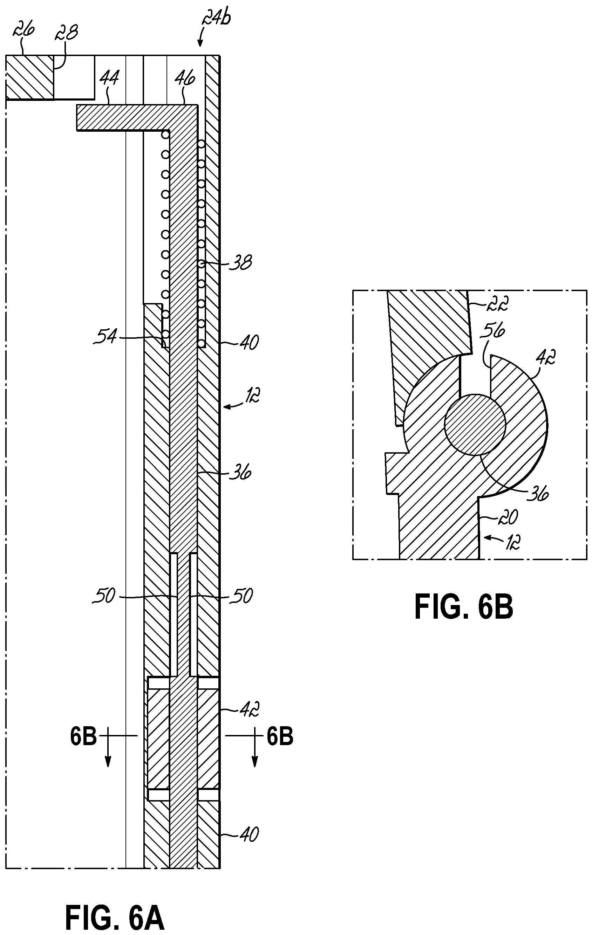

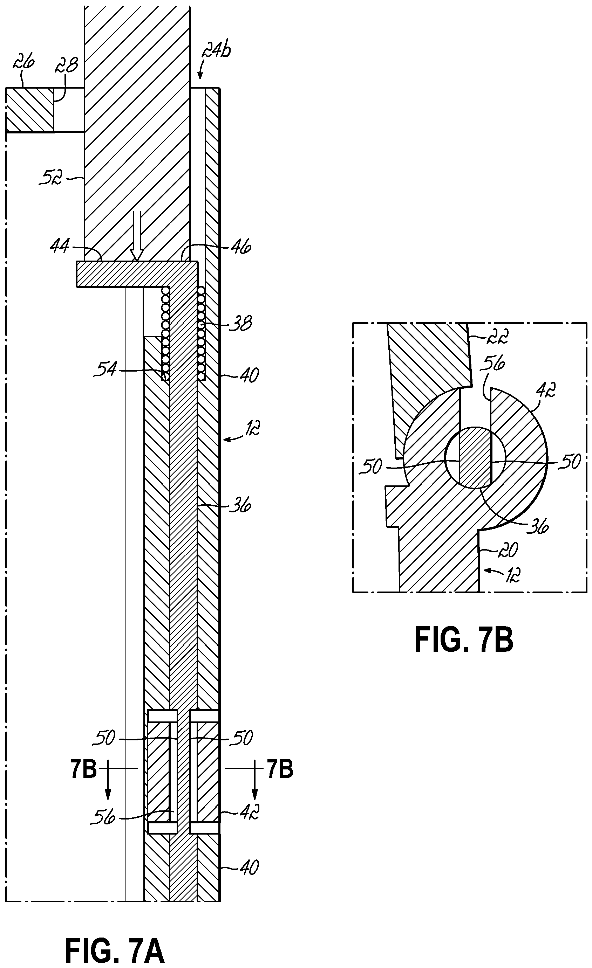

As shown in Fig. 3, each hinge rod 36 may include a number of spaced flats 50 along its length, three of which are shown in Fig. 3. The actuating member 44 at the uppermost end of each hinge rod 36 may include the connecting arm 46 connecting the member 44 to the upper end of the rod 36. The hinge spring 38 is mounted concentrically around the hinge rod 36 in the uppermost hinge barrel 40 to urge or bias the hinge rod 36 upwardly. The hinge spring 38 is seated in a hinge pocket 54 formed in the uppermost hinge barrel 40 as seen in Fig. 6A. The flats 50 are located in pairs which are diametrically opposite from each other as seen in Fig. 6A. The actuating member 44 is accessible to a user from the top of the dispenser 10 and when depressed downwardly with a tool 52 in one embodiment or by hand in another embodiment, the spring 38 compresses and the hinge rod 36 shifts downwardly within the hinge barrels 40 and the hinge collars 42. A tool or other device may be used to depress the actuating member 44 downwardly from locked configuration (Fig. 6A) to an unlocked configuration (Fig. 7A). When in the locked configuration, the fully round portions of the hinge rod 36 are seated in the hinge collars 42 as shown in Figs. 6A-6B allowing the door 20 to pivot about the hinge rod 36. When in the unlocked configuration, the flats 50 on the hinge rod 36 are positioned in the hinge collars 42 as shown in Figs. 7A-7B. Each hinge collar has a slot 56 sized and configured to allow the flats 50 on the hinge rod 36 to pass therethrough when in the unlocked configuration with the spring 38 compressed thereby allowing the door to pivot about the opposite hinge rod 36 to an open position as shown in Fig. 8.

Since each hinge assembly 24a, 24b may be actuated to open the door 20, the dispenser 10 may be mounted in a variety of positions while still allowing for the door 20 to be opened for re-stocking and/or servicing of the dispenser 10. In many settings, it is desirable to mount the dispenser 10 adjacent to a wall, partition or other structure which would make it difficult or inaccessible to open the door 20. Since dual hinge assemblies 24a, 24b are included in the dispenser 10 of various embodiments of this invention, the dispenser 10 may be mounted in any of a variety of settings without modification specialization. As shown in Fig. 8, the door 20 may be opened in the direction of arrows A about hinge assembly 24a when the actuating member 44 of hinge assembly 24b is depressed. Alternatively as shown in phantom lines of Fig. 8, the door 20 maybe opened about hinge assembly 24b when the actuating member of hinge assembly 24a is depressed. Moreover, the door 20 may be entirely removed from the housing 12 if both actuating members 44 of both hinge assemblies 24a, 24b are simultaneously depressed. The bi-directional hinging door 20 allows mounting of the dispenser 10 in bathrooms of all sizes and layouts.

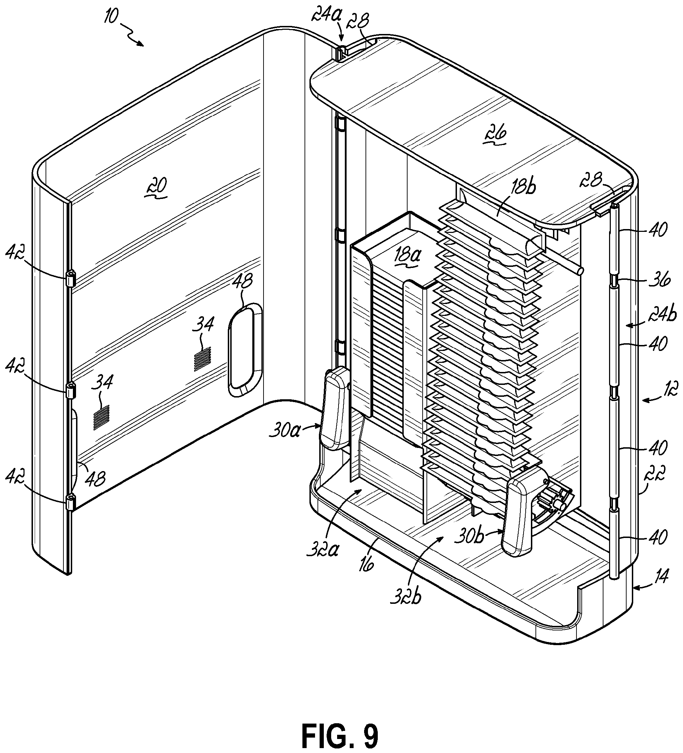

As shown in Fig. 9, the dispenser modules 32a, 32b may be mounted side by side within the housing 12 of the dispenser 10. Each module 32a, 32b may be similar to each other module 32a, 32b for dispensing similar products 18a, 18b or the modules 32a, 32b may be different for dispensing different products 18a, 18b as in Fig. 9. The module 32a on the left of the embodiment seen in Fig. 9 is designed to dispense pads 18a while the module 32b on the right is designed to dispense tampons 18b. Each module 32a, 32b will be described in detail hereinbelow.

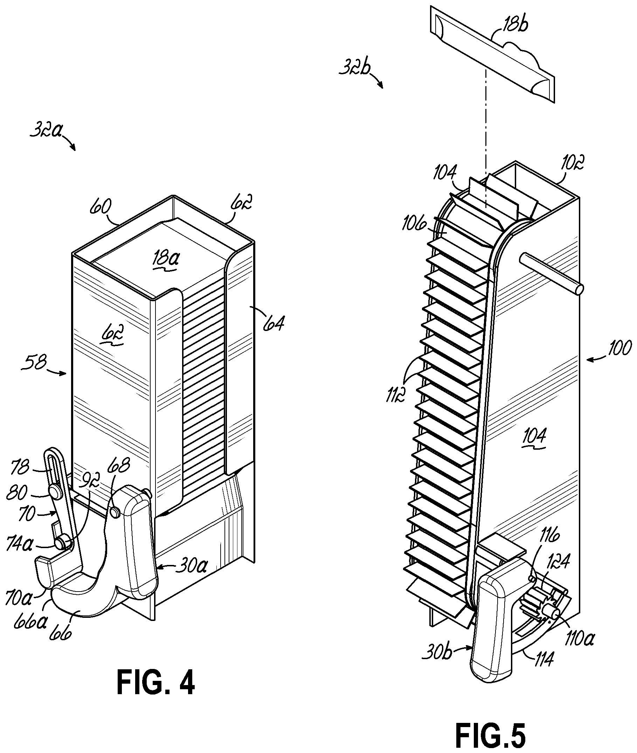

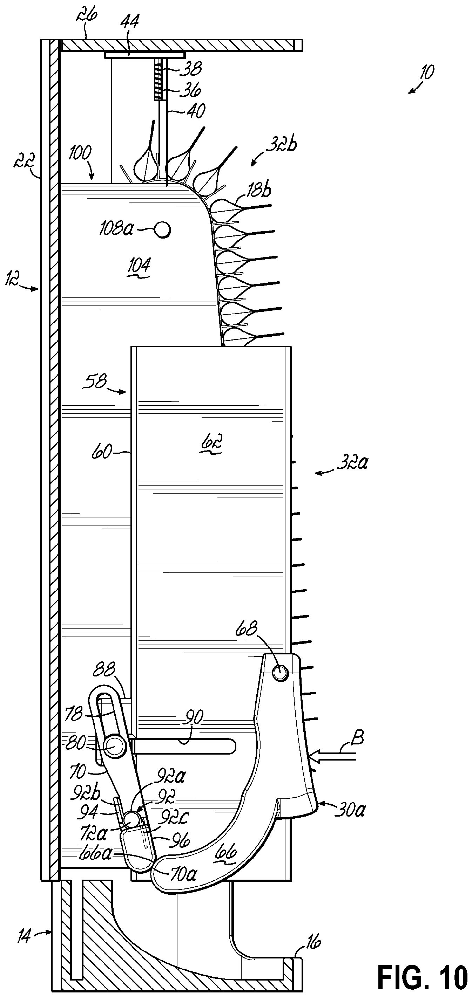

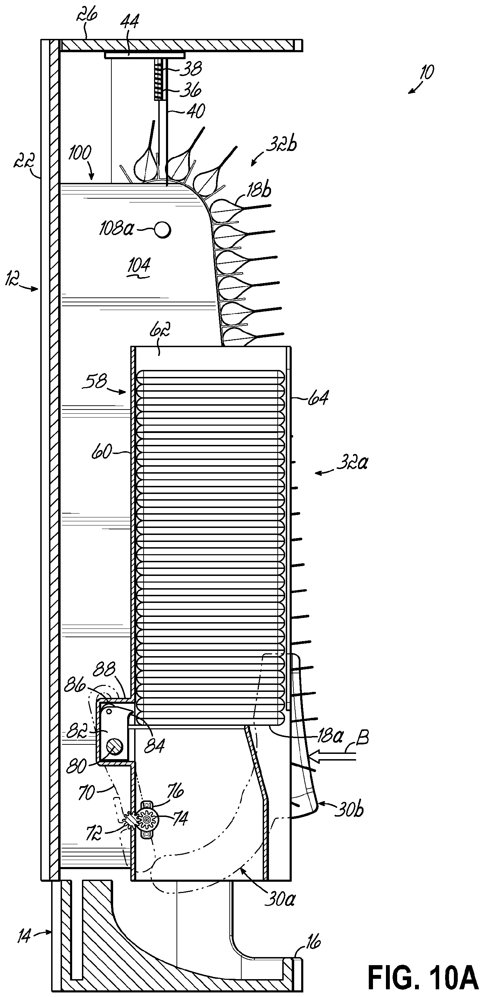

The dispenser module 32a for pad products 18a is shown in Figs. 10-11A and includes a three-sided casing 58 with a back wall 60 and opposite side walls 62, 62. A flange 64 projects inwardly from a terminal edge of each side wall 62 (Figs. 2 and 4). The casing 58 holds a supply of pads 18a stacked one atop of another and the bottom most pad 18b is the next product 18a, 18b to be dispensed from the module 32a by a dispensing mechanism when the button 30a of the dispensing mechanism vis depressed by a user. A curved leg 66 projects downwardly from the button 30a and the upper end of the button 30a is pinned to the adjacent casing sidewall 62a by a pivot pin 68 such that when the button 30a is depressed it pivots about the pin 68 and the leg 66 pushes inwardly toward the back wall 60 of the casing 58. A distal end 66a of the leg 66 is in contact with a lower end 70a of a link 70. As shown in Fig. 10A, the link 70 has a planetary gear 72 mounted thereto. The planetary gear 72 is engaged for rotation about a sun gear 74 mounted to a plate 76 fixed relative to the casing 58. A slot 78 is formed in the link 70 opposite from the end of the link 70 in contact with the leg 66. A pin 80 is captured in the slot 78 for movement therein. The pin 80 extends from a driver 82 having a forward projecting pick 84. A roller 86 is mounted atop the driver 82 for rotation. When the module 32a is at rest and not dispensing product 18a, the driver 82 is seated in a pocket 88 projecting rearwardly from the back wall 60 of the casing 58 as shown in Fig. 10A.

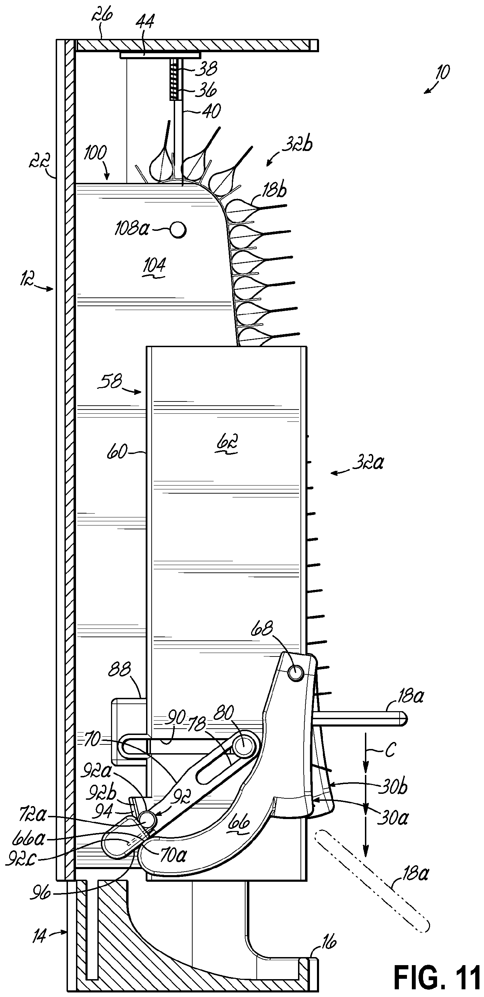

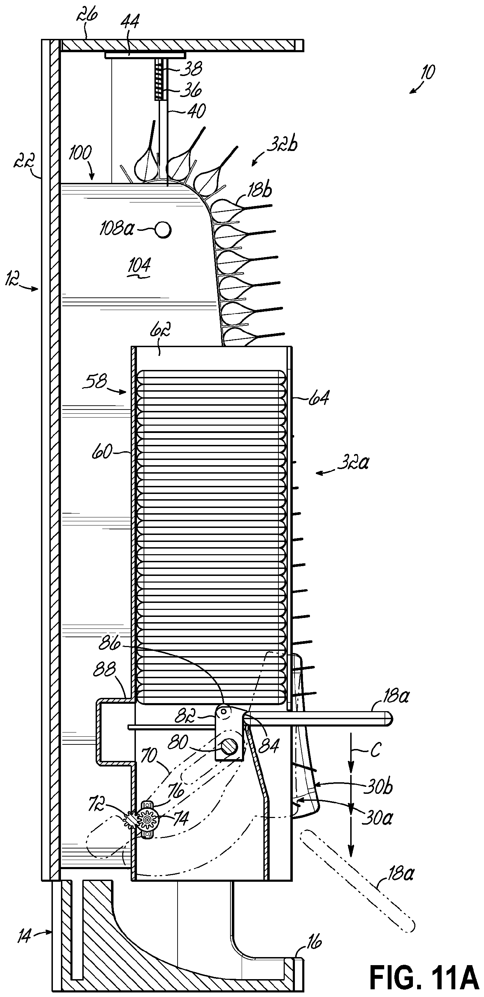

The dispensing operation for the module 32a begins with a user pushing the button 30a in the direction of arrow B in Fig. 10A. When the button 30a is pushed, the leg 66 moves rearwardly with the distal end 66a pushing the bottom end of the link 70 to pivot the link 70 clockwise as shown in Fig. 11. The link 70 pivots about the planetary gear 72 which meshes with the sun gear 74. When the link 70 pivots clockwise as seen in Fig. 11A, driver 82 is pushed forwardly from the pocket 88 so that the pick 84 contacts the back end of the product 18a. The driver 82 is urged forwardly by the movement of the link 70 with the pin 80 in the slot 78 of the link 70. A pusher slot 90 is in the sidewall 62 of the casing 58 to guide the pin 80 and driver 82 connected thereto as shown in Fig. 11. When the driver 82 approaches the forward extent of its travel and the pin 80 approaches the end of the slots 78 and 90, the bottom most product 18a is moved forwardly from the stack of product 18a, 18b and falls by gravity in the direction of arrows C into the tray 14 for retrieval by the user. The driver 82 is limited to linear movement because the pin 80 connected thereto is captured in the slot 90. The movement of the driver 82 forwardly is aided by the movement of the roller 86 holding the stack of product above the bottommost product 18a as shown in Fig. 11A. The friction between the driver 82 and the next lowest product 18a is reduced/minimized by the roller 88 rolling along the lower face of the next lowest product 18a.

Once the product 18a is dispensed from the module 32a, the button 30a is pushed outwardly to a ready position, the driver 82 is returned to the pocket 88 and the link 70 is rotated counterclockwise to the position shown in Fig. 10 by a torsion spring 92 with a helical body 92a mounted concentrically around planetary gear pivot pin 72a and a first arm 92b abutting against a detent 94 on the casing 58 and a second arm 92c abutting against a detent 96 on the link 70. The torsion spring 92 biases the components of the module 32a toward the ready position shown in Fig. 10.

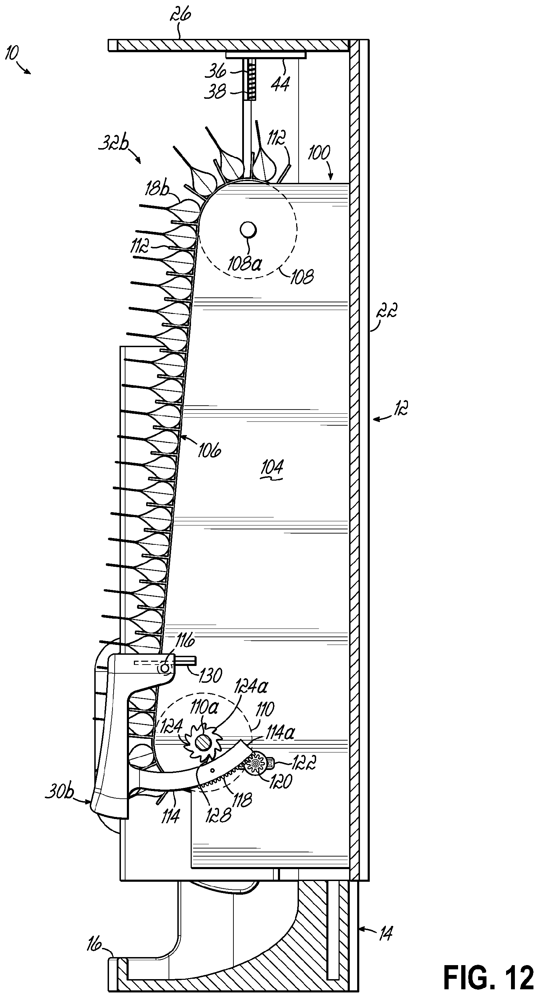

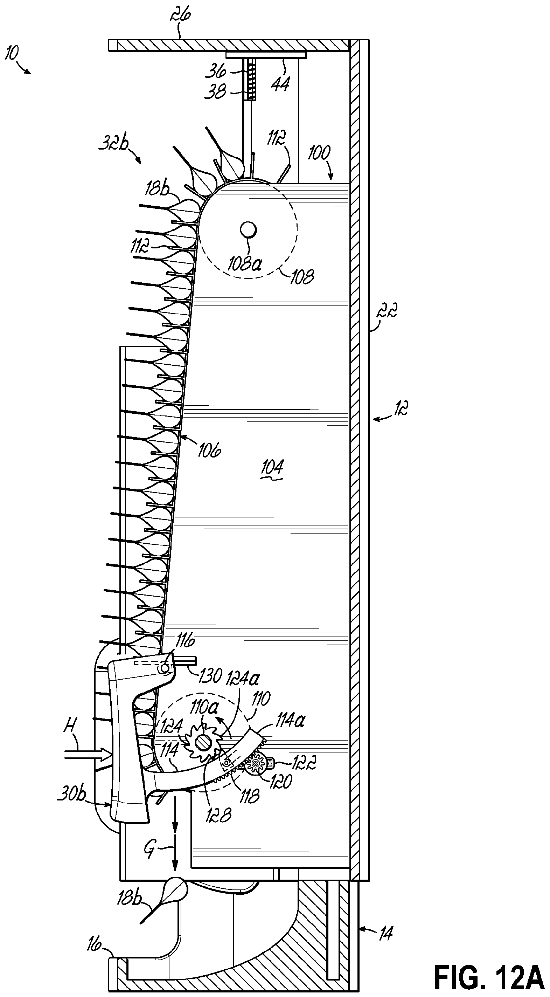

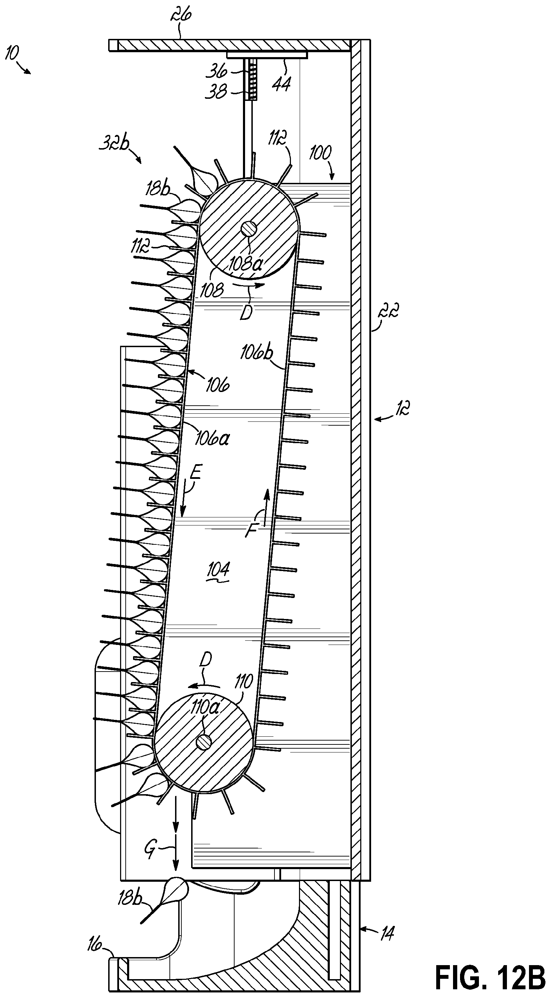

The dispenser 10 may include a second dispensing module 32b adapted to dispense individual tampon products 18b via a dispensing mechanism. One embodiment of the dispensing module 32b is shown in Figs. 2, 5, 9, and 12-12B. The dispensing module 32b includes a three-sided casing 100 with a back wall 102 and opposite side walls 104, 104. A belt 106 is trained around an upper pulley 108 and a lower pulley 110 as shown in Fig. 12. The upper pulley 108 is longitudinally offset from the lower pulley 110 such that a forward portion 106a of the belt 106 is inclined at an angle A relative to a vertical plane as shown in Fig. 12B. The pulleys 108, 110 are mounted on rods 108a, 110a, respectively, for rotation in the direction of arrows D. When the pulleys 108, 110 rotate in the direction of arrows D, the forward portion 106a of the belt 106 moves downwardly in the direction of arrow E and a rearward portion 106b of the belt 106 moves upwardly in the direction of arrow F.

The belt 106 has a number of cleats 112 projecting from an outer surface of the belt 106. Individual products 18b are seated between adjacent cleats 112 and the outer face of the belt 106. The products 18b are located primarily on the forward portion 106a of the belt 106 and rest on the associated lower cleat 112 due in part the inclination of the forward portion 106a. As the belt 106 advances in the direction of arrow F and around the lower pulley 110, the lowermost product 18b falls from the belt 106 in direction of arrow G onto the tray 14.

To dispense a product 18b from the module 32b, a user pushes the button 30b in the direction of arrow H in Fig. 12A. A curved leg 114 projects rearwardly from the button 30b and the upper end of the button 30b is pinned to the adjacent casing sidewall 104 by a pivot pin 116 such that when the button 30b is depressed it pivots about the pin 116 and the leg 114 pushes inwardly toward the back wall 60 of the casing 58. A distal end 114a of the leg 114 has a number of teeth 118 projecting downwardly to mesh with a rotary dispensing gear 120 mounted to a plate 122. The dispensing gear 120 is part of a dampening assembly for the dispensing of the products 18b in the module 32b. A ratchet gear 124 is mounted on a rod 110a for the lower pulley 110 as shown in Fig. 12. A pawl 128 projects upwardly from the curved leg 114 and engages one of the teeth 124a of the ratchet gear 124 each time the button 30b is pushed. The pawl 128 is pinned to the curved leg 114 to pivot rearwardly but is rigid in the forward direction.

When the curved leg 114 moves rearwardly in response to the button 30b being pushed, the pawl 128 engages one of the teeth 124a on the ratchet gear 124 to thereby rotate the lower pulley 110 and the upper pulley 108 a desired amount to advance the belt 106 and deposit the lowermost product 18b into the tray 14. The teeth 118 on the curved leg 114 mesh with the teeth on the rotating dispensing gear 120 to provide a dampening effect on the operation. A torsion spring 130 is mounted on the pivot pin 116 to urge the button 30b outwardly once it is released by a user and a product 18b has been deposited into the tray 14. When the button 30b is released, the spring 130 pushes the button 30b and the curved leg 114 out and the pawl 128 disengages from the ratchet gear 124. The one-way damped return of the button 30b and associated components offers a refined feel and prevention of rapid dispensing.

Each of the modules 32a, 32b offer smooth acting generously sized buttons 30a, 30b for single product 18a, 18b dispensing onto the open, shared tray to increase ease of cleaning and an angled access area to present and position product 18a, 18b for easy retrieval. The dispensing mechanisms for the modules 32a, 32b offer generous touch points to enable activation flexibility to reduce hand contact.

Viewing arrangements in the form of slots 34 are provided in the door 20 to communicate product level in the modules 32a, 32b.

Embodiments of the disclosure can be described with reference to the following numbered clauses, with additional features laid out in the dependent clauses:
Clause 1. A feminine hygiene product dispenser comprising:
   a housing;
   a door on the housing which is selectively opened and closed to allow for access and prevent access, respectively, to an interior of the housing; and
   a first dispensing module and a second dispensing module in the housing, the first and second dispensing modules being operable by a user to dispense a first and a second feminine hygiene product, respectively, from the housing;
   wherein the first and second dispensing modules are different from each other and the first and second feminine hygiene products are different from each other.
Clause 2: The dispenser according to clause 1 further comprising:
   a product retrieval tray onto which the first and second dispensing modules deposit the first and second feminine hygiene products, respectively, for retrieval by the user.
Clause 3: The dispenser according to clause 1 or 2, wherein the door is hingedly coupled to the housing for pivotal movement.
Clause 4: The dispenser according to any one of clauses 1 to 3, wherein the door can be opened to first and second opened positions which are different one from another.
Clause 5: The dispenser according to clause 3 further comprising:
   a first and a second hinge assembly each coupling the door to the housing;
   wherein the door is selectively pivotal about the first hinge assembly to a first open position and the door is selectively pivotal about the second hinge assembly to a second open position which is different from the first open position.
Clause 6: The dispenser according to clause 5, wherein the first and second hinge assemblies are spaced from each other on opposite sides of the door and each are oriented generally vertically.
Clause 7: The dispenser according to clause 5 or 6, further comprising:
   a first and a second actuating member each of which is selectively actuated by the user to release the door to pivot about the second and the first hinge assembly, respectively.
Clause 8: The dispenser according to clause 7, wherein the first and second actuating members may be simultaneously activated to remove the door from the housing.
Clause 9: The dispenser according to any preceding clause, wherein the first dispensing module further comprises:
   a casing adapted to hold a plurality of the first feminine hygiene products in a vertical stack therein;
   a dispensing mechanism adapted to dispense a lowermost one of the plurality of the first feminine hygiene products from the vertical stack; and
   a button which actuates the dispensing mechanism when depressed by the user.
Clause 10: The dispenser according to any preceding clause, wherein the second dispensing module further comprises:
   a belt having a plurality of spaced cleats and adapted to hold a plurality of the second feminine hygiene products;
   a dispensing mechanism adapted to dispense a lowermost one of the plurality of the second feminine hygiene products from the belt; and
   a button which actuates the dispensing mechanism when depressed by the user.
Clause 11: The dispenser according to any preceding clause, further comprising:
   a viewing arrangement in one of the housing and the door through which the user may determine the presence of at least one of the first and the second feminine hygiene products therein.
Clause 12: A feminine hygiene product dispenser comprising:
   a housing;
   a door hingedly coupled to the housing for pivotal movement to selectively allow for access and prevent access to an interior of the housing; and
   a first dispensing module in the housing being operable by a user to dispense a first feminine hygiene product from the housing;
   wherein the door can be opened to first and second opened positions which are different one from another.
Clause 13: The dispenser according to clause 12 further comprising:
   a first and a second hinge assembly each coupling the door to the housing;
   wherein the door is selectively pivotal about the first hinge assembly to a first open position and the door is selectively pivotal about the second hinge assembly to a second open position which is different from the first open position.
Clause 14: The dispenser according to clause 13 wherein the first and second hinge assemblies are spaced from each other on opposite sides of the door and each are oriented generally vertically.
Clause 15: The dispenser according to clause 14 or 15, further comprising:
   a first and a second actuating member each of which is selectively actuated by the user to release the door to pivot about the second and the first hinge assembly, respectively.
Clause 16: The dispenser according to clause 15 wherein the first and second actuating members may be simultaneously activated to remove the door from the housing.
Clause 17: A method for selectively supplying feminine hygiene products to a user comprising the steps of:
   storing a first plurality of feminine hygiene products in a first dispenser module in a dispenser housing;
   storing a second plurality of feminine hygiene products in a second dispenser module in the dispenser housing;
   selectively closing the dispenser housing with a door to limit access to the first and second feminine hygiene products in the dispenser housing;
   engaging a first member coupled to the first dispenser module to dispense one of the first plurality of feminine hygiene products from the dispenser housing; and
   engaging a second member coupled to the second dispenser module to dispense one of the second plurality of feminine hygiene products from the dispenser housing.
Clause 18: The method according to clause 17 further comprising:
   stacking the first plurality of feminine hygiene products in a generally vertical stack; and
   pushing a lowermost one of the first plurality of feminine hygiene products from the generally vertical stack toward an outlet of the dispenser housing.
Clause 19: The method according to clause 17 or 18, further comprising:
   supporting each of the second plurality of feminine hygiene products on a belt and one of a plurality of cleats extending from the belt; and
   advancing the belt to deposit a lowermost one of the second plurality of feminine hygiene products therefrom toward an outlet of the dispenser housing.
Clause 20: The method according to any one of clauses 17 to 19, further comprising:
   pivotally coupling the door to the housing about a first hinge assembly for movement of the door to first open position;
   pivotally coupling the door to the housing about a second hinge assembly for movement of the door to second open position which is different from the first open position.
From the above disclosure of the general principles of this invention and the preceding detailed description of at least one embodiment, those skilled in the art will readily comprehend the various modifications to which this invention is susceptible. Therefore, we desire to be limited only by the scope of the following claims and equivalents thereof.

## Claims

1. A feminine hygiene product dispenser comprising:
a housing;
a door on the housing which is selectively opened and closed to allow for access and prevent access, respectively, to an interior of the housing; and
a first dispensing module and a second dispensing module in the housing, the first and second dispensing modules being operable by a user to dispense a first and a second feminine hygiene product, respectively, from the housing;
wherein the first and second dispensing modules are different from each other and the first and second feminine hygiene products are different from each other.

2. The dispenser according to claim 1 further comprising:
a product retrieval tray onto which the first and second dispensing modules deposit the first and second feminine hygiene products, respectively, for retrieval by the user.

3. The dispenser according to claim 1 or 2, wherein the door is hingedly coupled to the housing for pivotal movement.

4. The dispenser according to any preceding claim, wherein the door can be opened to first and second opened positions which are different one from another.

5. The dispenser according to claim 3 further comprising:
a first and a second hinge assembly each coupling the door to the housing;
wherein the door is selectively pivotal about the first hinge assembly to a first open position and the door is selectively pivotal about the second hinge assembly to a second open position which is different from the first open position.

6. The dispenser according to claim 5 wherein the first and second hinge assemblies are spaced from each other on opposite sides of the door and each are oriented generally vertically.

7. The dispenser according to claim 5 or 6, further comprising:
a first and a second actuating member each of which is selectively actuated by the user to release the door to pivot about the second and the first hinge assembly, respectively.

8. The dispenser according to claim 7, wherein the first and second actuating members may be simultaneously activated to remove the door from the housing.

9. The dispenser according to any preceding claim, wherein the first dispensing module further comprises:
a casing adapted to hold a plurality of the first feminine hygiene products in a vertical stack therein;
a dispensing mechanism adapted to dispense a lowermost one of the plurality of the first feminine hygiene products from the vertical stack; and
a button which actuates the dispensing mechanism when depressed by the user.

10. The dispenser according to any preceding claim, wherein the second dispensing module further comprises:
a belt having a plurality of spaced cleats and adapted to hold a plurality of the second feminine hygiene products;
a dispensing mechanism adapted to dispense a lowermost one of the plurality of the second feminine hygiene products from the belt; and
a button which actuates the dispensing mechanism when depressed by the user.

11. The dispenser according to any preceding claim, further comprising:
a viewing arrangement in one of the housing and the door through which the user may determine the presence of at least one of the first and the second feminine hygiene products therein.

12. A method for selectively supplying feminine hygiene products to a user comprising the steps of:
storing a first plurality of feminine hygiene products in a first dispenser module in a dispenser housing;
storing a second plurality of feminine hygiene products in a second dispenser module in the dispenser housing;
selectively closing the dispenser housing with a door to limit access to the first and second feminine hygiene products in the dispenser housing;
engaging a first member coupled to the first dispenser module to dispense one of the first plurality of feminine hygiene products from the dispenser housing; and
engaging a second member coupled to the second dispenser module to dispense one of the second plurality of feminine hygiene products from the dispenser housing.

13. The method according to claim 12, further comprising:
stacking the first plurality of feminine hygiene products in a generally vertical stack; and
pushing a lowermost one of the first plurality of feminine hygiene products from the generally vertical stack toward an outlet of the dispenser housing.

14. The method according to claims 12 or 13, further comprising:
supporting each of the second plurality of feminine hygiene products on a belt and one of a plurality of cleats extending from the belt; and
advancing the belt to deposit a lowermost one of the second plurality of feminine hygiene products therefrom toward an outlet of the dispenser housing.

15. The method according to any one of claims 12 to 14, further comprising:
pivotally coupling the door to the housing about a first hinge assembly for movement of the door to first open position;
pivotally coupling the door to the housing about a second hinge assembly for movement of the door to second open position which is different from the first open position.
